# EUROPEAN PATENT SPECIFICATION

(11) **EP 0 528 971 B1**
(45) Date of publication and mention of the grant of the patent: **01.09.1999**
(21) Application number: 91910569.2
(22) Date of filing: 23.04.1991
(51) Int. Cl.: A61K 9/22, A61K 9/52, A61L 27/00

(54) **SURGICAL IMPLANT AND METHOD INCORPORATING CHEMOTHERAPEUTIC AGENTS**
CHIRURGISCHES IMPLANTAT UND VERFAHREN ZUM EINBETTEN VON CHEMOTHERAPEUTIKA
PROCEDE ET IMPLANT CHIRURGICAL UTILISANT DES AGENTS CHIMIOTHERAPEUTIQUES

(30) Priority: 14.05.1990 US 523067; 18.10.1990 US 599699
(43) Date of publication of application: 03.03.1993
(73) Proprietor: JERNBERG, Gary R., Mankato, Minnesota 56001 (US)
(72) Inventor: JERNBERG, Gary R., Mankato, Minnesota 56001 (US)
(74) Representative: Petri, Stellan
(86) International application number: US9102784
(87) International publication number: WO9117744

(56) References cited:
- EP-A- 0 216 453
- EP-A- 0 293 090
- EP-A- 0 406 665
- WO-A-87/06129
- GB-A- 2 033 232
- GB-A- 2 103 927

## Description

### Technical Field of the Invention

The present invention relates to controlled release and improved cellular uptake of chemotherapeutic agents over a predetermined period of time.

### Background of the Invention

Biologically compatible materials capable of being formed into implants are increasing in use in surgery and medicine. Examples include vascular grafts, ligament prostheses and reconstructive patches.

Utilization of surgical implants presents several problems to the practitioner. For example, the potential of infections exists with surgically placed implants, including grafts, prostheses, etc. See e.g., E. J. Young and B. Sugarman, Infections in Prosthetic Devices, 68 Surg.Clin. N. Am., 167 (1988); The International Journal of Periodontics and Restorative Dentistry, April (1988); B. Van der Lei, et al., Expanded Polytetrafluoroethylene Patch for the Repair of Large Abdominal Wall Defects 76 Br. J.Surg., 803 (1989); N. S. Horbach, et al., A Suburethral Sling Procedure with Polytetrafluoroethylene for the Treatment of Genuine Stress Incontinence in Patients with low Urethral Closure Pressure, 71 Obstete. Gynecol., 648 (1988).

Adverse clotting problems can also occur with vascular grafts. N. A. Shoenfeld, et al., A New Primate Model for the Study of Intravenous Thrombotic Potential and its Modification, 8 J. Vasc. Surg., 49 (1988); L. J. Dacey, et al., Intraarterial 9-beta-methyl carbacyclin Improves Canine Polytetrafluoroethylene Graft Patency, 8 J. Vasc. Surg., 21 (1988).

Potential infection and excessive inflammation can create problems with orthopedic prostheses. See e.g., E. J. Young and B. Sugarman, supra; S. P. F. Hughes, Treatment of Infected Implants, Antibiotic Acrylic Composites, 16 Orthopaedic Review, 233 (1987); J. H. Roth, et al., Synovial Reaction Associated with Disruption of Polypropylene Braid-augmented Intraarticular Anterior Cruciate Ligament Reconstruction, 16 Am.J. Sports Med., 301 (1988); S. K. Ahlfeld, et al., Anterior Cruciate Reconstruction in the Chronically Unstable Knee using an Expanded Polytetrafluoroethylene (PTFE) Prosthetic Ligament, 15 Am.J. Sports Med., 326 (1987).

Accordingly, the *"*take*"* of these implants, such as a cruciate ligament prosthesis, is variable.

Chemotherapeutic agents have been previously incorporated into vascular grafts. For example, U.S. Patent No. 4,321,711 discloses a vascular prosthesis comprising porous tubing of polytetrafluoroethylene containing an anti-coagulant substance with a porous elastomer coating, containing a substance which counteracts the anti-coagulant, bonded to its outside surface . Typically, the anti-coagulant substance is heparin. Any heparin antagonist such as protamine may be used in the elastomer coating to counteract the heparin. U.S. Patent No. 4,816,339 also refers to the use of therapeutically active substances, such as heparin or antibiotics, placed into an elastomer solution which surrounds a luminal polytetrafluoroethylene layer. However, the incorporated chemotherapeutic agents are soon exhausted from these implants, resulting in renewed potential for clotting. Thus, neither of these inventions provide for the sustained, controlled release of chemotherapeutic agents, nor the enhanced cellular uptake of these agents, that the present invention would provide.

WO-A-87/06129 describes a sustained-release implant which has a biodegradable polymeric article, a plurality of biodegradable microcapsules embedded therein, and an effective amount of a physiologically active ingredient and capsulated in the microcapsules. The biodegradable implant is described as suitable for forming into a projectile to be delivered into the body of a wild animal by using, for example, an air gun.

US-A-3 618 604 describes an ocular insert constructed of a flexible polymeric material having an imperforate surface. The ocular insert is adapted for insertion beneath the lower lid of an eye and is held in place by the pressure of the lid. When the insert is inserted in an eye, a drug contained in the insert is dispensed to the eye by diffusion through the polymeric material.

US-A-3 960 150 describes an ocular insert which functions as a drug delivery device. The ocular insert includes a bio-erodible matrix having dispersed therethrough a plurality of microcapsules comprised of a drug. When the ocular insert is inserted into the eye of a patient, the bio-erodible matrix bio-erodes thereby delivering the drug to the eye.

US-A-4 321 711 relates to a vascular prosthesis containing an anti-coagulant and having an elastomer coating containing a substance for counteracting the anti-coagulant.

US-A-4 685 883 describes a method for delivery of a chemotherapeutic agent to a localized site in the mouth of a patient. The method includes insertion of a biodegradable time-release microsphere encapsulating the therapeutic agent into the gingival tissues of the patient. As the time-release microsphere biodegrades, the chemotherapeutic agent is continuously delivered over a predetermined period of time.

US-A-4 780 320 describes a controlled-release drug delivery system for placement in the periodontal pocket. The system includes a purality of drug-containing microparticles suspended in a carrier medium. The carrier medium is introduced into the periodontal pockets. While in the periodontal pockets, the drug-containing microparticles biodegrade thereby continuously delivering the drug to the periodontal pocket.

US-A-4 837 285 describes a collagenbased composition for augmenting soft tissue repair. The composition includes resorbable collagen beads having a matrix being sufficiently open to stimulate cellular ingrowth and sufficiently stiff to fill and protect a wound.

US-A-4 851 521 describes medicaments containing a pharmacologically active substance in combination with a carrying vehicle containing a total or partial ester of hyaluronic acid.

### Summary of the Invention

The present invention solves the problems referred to above and many other problems associated with surgical implantation, successful grafting and tissue regeneration by providing a surgical implant comprising a nonresorbable biocompatible matrix with controlled, sustained release microshapes encapsulating at least one chemotherapeutic agent incorporated into the matrix, wherein the time-release microshapes will begin to release said chemotherapeutic agent at a localized treatment sight upon surgical implantation of the implant.

Accordingly, an advantage of one embodiment of the present invention is to provide vascular grafts incorporating microencapsulated chemotherapeutic and optional carrier agents which upon implantation provide for sustained, controlled delivery and improved cellular uptake of anticoagulant agents at the implantation site such that the formation of blood clots (thrombi) are prevented. In addition, microencapsulated chemotherapeutic agents which act as an antagonist to the anticoagulant may be incorporated into an outer layer of the vascular graft.

An advantage of yet another embodiment is to provide prostheses incorporating microencapsulated chemotherapeutic agents and optional carrier agents, including for example, cruciate ligament prostheses, which upon implantation provide for sustained, controlled delivery and improved cellular uptake of antibiotic, anti-inflammatory and other appropriate agents to the implantation site, such that the retention of said prostheses is improved.

These and various other advantages and features of novelty which characterize the invention are pointed out with particularity in the claims annexed hereto and forming a part hereof. However, for a better understanding of the invention, its advantages, and objects attained by its use, reference should be had to the drawings which form a further part hereof, and to the accompanying descriptive matter, in which there is illustrated and described a preferred embodiment of the invention.

### Brief Description of the Drawings

In the drawings, in which like reference numerals and letters indicate corresponding parts throughout the several views,
Figures 1A through 1D are diagrammatic perspective views of alternate configurations of microparticles which might be used in the present invention to contain chemotherapeutic agents or carrier agents;
Figures 2A through 2C are diagrammatic sectional views of alternate embodiments of microparticles having a somewhat spherical configuration with outside walls of varying thicknesses so as to provide for different timed release of chemotherapeutic or carrier agents from inside the microparticles;
Figure 3 illustrates an example of a prior art vascular graph generally according to U.S. Patent No. 4,321,711;
Figures 4A through 4D are end sectional views of vascular grafts illustrating alternate embodiments of vascular grafts showing various methods of incorporation of the microparticles into the vascular grafts;
Figure 5 illustrates an embodiment of a cruciate ligament prosthesis incorporating microencapsulated chemotherapeutic and optional carrier agents in accordance with the principles of the present invention;
Figures 6A,B are an enlarged sectional view of a fiber bundle of the ligament prosthesis shown in Figure 5, illustrating incorporation of the microencapsulated chemotherapeutic and optional carrier agents;
Figures 7A through 7D illustrate representative time release patterns of various chemotherapeutic agents which might be obtained by the appropriate microparticle configuration, microparticle arrangement in the implant, and chemotherapeutic agents used.

### Detailed Description of the Invention

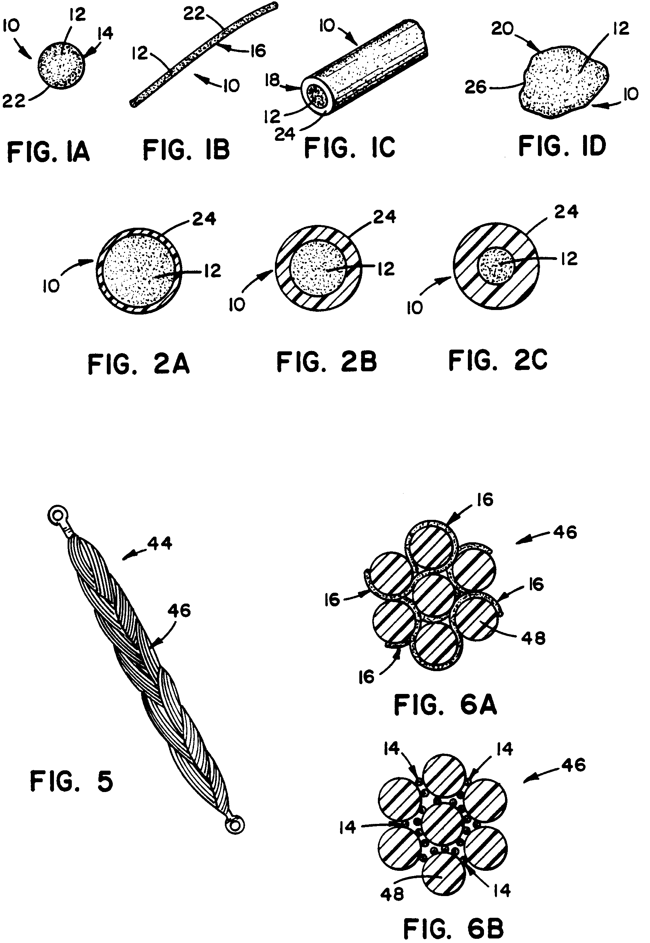

Referring now to Figures 1A-1D, time-release microparticles 10 containing chemotherapeutic and optional carrier agents 12, and incorporated into the matrix of the implants, including, but not limited to, grafts, prostheses, etc., of the present invention can occur in a variety of shapes and sizes.

The microparticles shown in Figures 1A-1D are greatly enlarged, and in actual use, might typically be less than a millimeter in size. As used herein, microparticles broadly include, without limitation, microspheres 14 (hollow and nonhollow), microfibers or microfibrils 16, hollow microfibers 18, microsponges 20, as well as any other microshape which incorporate chemotherapeutic and optional carrier agents into their body or matrix. An outer shell 22 of the microspheres 14 and the microfibers 16, an outer wall 24 of the hollow microspheres 14 and hollow microfibers 18, or matrix 26 of the microsponge 20 is composed of a biodegradable material, thereby allowing for the controlled, sustained release and improved cellular uptake of the chemotherapeutic agents over time.

In one embodiment, the microparticles are incorporated into the microstructure of the material comprising an implant according to the present invention. For example, the microspheres 14 can be contained within the mesh of fine fibrils connecting the matrix of nodes in expanded polytetrafluoroethylene (PTFE). In addition, somewhat larger microspheres can be meshed between layers of a multi-layered PTFE implant structure. The microspheres can be layered within the PTFE implant by adhesively positioning them onto the PTFE or by mixing them with a fluid and/or gel and flowing them into the netting or weave of the material. In such an embodiment, the fluid and gel can be carrier agents such as hyaluronic acid and a cross-linked gel of hyaluronic acid respectively. Finally, microspheres can also be positioned between the implant and an elastomer coating covering said implants.

In another embodiment, the microfibers or microfibrils can be woven into the mesh of the implant or, as described above, layered between successive layers of PTFE, or a similar material, comprising the implant.

In yet another embodiment, the microparticles may be in the form of microsponges which contain the desired chemotherapeutic and optional carrier agents within their microchanneling.

Microspheres between 10 and 700 microns in diameter are preferred. Various chemical and physical methods for preparing microspheres have been developed over the past twenty-five years and are well known to those skilled in the art. In this regard, see for example Patrick B. Deasy, Microencapsulation and Related Drug Processes. Marcel Dekker Inc., New York, 1984. Coacervation, interfacial polymerization, solvent evaporation and spray drying are examples of methods used in the production of microspheres which incorporate chemotherapeutic and optional carrier agents. Similarly, microfibers or microfibrils can be obtained for layering or weaving into the implant materials of the present invention. In this regard, hollow microfibers ranging in size from 100 to 1,000 microns in diameter can be produced and drug loaded by extrusion.

A wide variety of chemotherapeutic agents can be incorporated into the microshapes employed according to the method of the present invention. For example, antibacterial agents such as the bisbiguanides, antibiotics such as vancomycin or tetracycline, anti-inflammatory agents such as indomethacin or hydrocortisone, anticoagulants such as heparin and tissue regenerative agents such as fibronectin may be employed, depending upon the particular treatment or preventative goals sought.

Incorporation of the chemotherapeutic and optional carrier agents into the polymer comprising the microshape provides for a slow, sustained release and enhanced cellular uptake of the chemotherapeutic agent. The polymer matrix or carrier material chosen is preferably biodegradable, pharmaceutically acceptable and available in various grades to allow for variable control of the release rate of different chemotherapeutic agents. In this regard, it will be appreciated that the biodegradable materials utilized in time release capsules taken orally or other suitable biodegradable materials safe for use in the body or commonly known may be employed. For example, various biocompatible polymers can be employed, including but not limited to, collagen, cellulosic polymers, ethylene vinyl acetate, methacrylate polymers, lactic-glycolic acid copolymers, polycaprolactone, etc. In addition, polymers such as polystyrene, polycarbonate, polysulfone, polylactides and polyurethane can be employed. It will be appreciated that nonbiodegradable polymers incorporating chemotherapeutic agents are also within the scope of the present invention.

Carrier agents to improve cellular uptake of chemotherapeutic agents can also be incorporated into the implant. In one embodiment, the carrier agents can be mixed with the chemotherapeutic agents for delivery by the microparticles in the previously mentioned configurations. In another embodiment, the carrier agents can be separately incorporated into microparticles, which are then combined with the microparticles incorporating the chemotherapeutic agents. In yet another embodiment, the carrier agents can be in the form of a fluid or gel positioned within the weave or netting of the implant. In still another embodiment, a cross-linked, polymerized form of the carrier agent can be utilized to form the body of the implant.

Preferred carrier agents include, without limitation, hyaluronic acid, salts thereof such as sodium hyaluronate, esters, ethers, enzymatic derivatives and cross-linked gels of hyaluronic acid and chemically modified derivatives of hyaluronic acid such as hylan. As used herein, hyaluronic acid broadly refers to naturally occurring, microbial and synthetic derivatives of acidic polysaccharides of various molecular weights constituted by residues of D-glucuronic acid and N-acetyl-D-glucosamine.

Referring now to Figures 2A-2C, wherein is illustrated diagrammatic sectional views of alternative embodiments of microspheres in accordance with the present invention. The microspheres 10 have a polymer wall or shell 24 which surrounds the chemotherapeutic and optional carrier agents 12, or matrix containing the chemotherapeutic and optional carrier agents. Thus, the walls of the microspheres may have varying thicknesses and/or be made of a different material to provide for release of the agent continuously or periodically over an extended time period following surgical placement of the implant. For example, an implant may contain an antibiotic which would be released from one type of microparticle during the first critical days following graft placement, whereas an anti-inflammatory agent contained in a second type of microparticle would be released several weeks after implantation. In addition, it is to be understood that the chemotherapeutic and optional carrier agents contained within the microsphere may occur in any appropriate medium, such as aqueous, gelatinous, colloidal or semi-solid media. Furthermore, a carrier agent may also be provided with the microencapsulated chemotherapeutic agent to enhance the cellular uptake of the chemotherapeutic agent at the desired treatment site.

In another embodiment according to the method of the present invention, the chemotherapeutic and optional carrier agents are positioned at strategic areas of the implants relative to their intended function. For example, an anticoagulant substance could be positioned within the body or internal lining of a vascular graft, while a substance counteracting the anticoagulant would be positioned at the graft outer surface. In addition, a carrier agent can be included to enhance the cellular uptake of the anticoagulant and its antagonist. In a further modification, other chemotherapeutic agents can be positioned at different strategic areas of the implant materials relative to their intended uses. For example, an antibiotic, alone or in conjunction with a carrier agent, could be positioned near the attachment points of a ligament prosthesis, while an anti-inflammatory agent could be positioned within the body of the prosthesis. Referring now to FIG. 3A, which illustrates a prior art drawing of a vascular graft according to U.S. Patent No. 4,321,711 (Mano). The Mano vascular graft 28 incorporates chemotherapeutic agents, such as the anticoagulant heparin, directly into the body of the vascular graft 30, while incorporating a counteracting substance to the anticoagulant in the elastomer coating surrounding the body of the vascular graft 32.

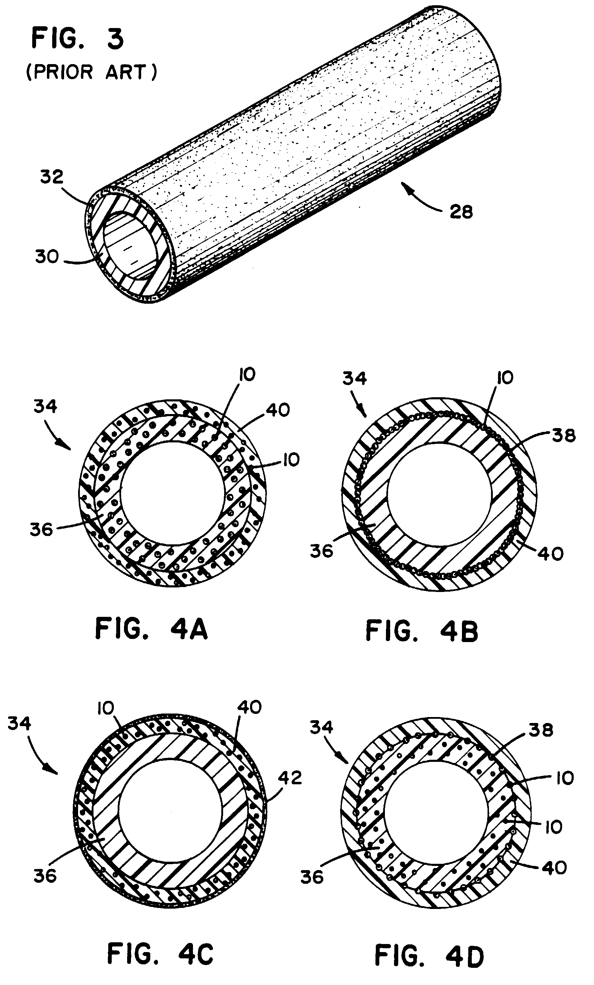

In comparison, the vascular graft of the present invention may incorporate microencapsulated chemotherapeutic and optional carrier agents into the body of the vascular graft, between the layers comprising the graft or in an elastomer coating surrounding the body of the graft. Thus, in Figures 4A-4D there is illustrated cross-sectional views of vascular grafts 34 incorporating microencapsulated chemotherapeutic and optional carrier agents 10 in accordance with the method and implant of the present invention. In particular Figure 4A depicts a cross-sectional view of a vascular graft 34 according to the method and implant of the present invention, wherein one microencapsulated chemotherapeutic agent 10, such as an anticoagulant, is incorporated into the inner layer of the graft 36. Conversely, another microencapsulated chemotherapeutic agent 10, such as an anticoagulant antagonist, is incorporated into the outer layer of the graft 40. Alternatively, as illustrated in Fig. 4B, a single incorporation of microencapsulated chemotherapeutic agents 10 may be placed between the layers comprising the graft 38. Figs. 4C-4D illustrate further potential embodiments. For example, in Fig. 4C, microencapsulated chemotherapeutic agents 10 are only incorporated into the outer layer 40 of a vascular graft 34 in accordance with the present invention, whereas in Fig. 4D, a first microencapsulated chemotherapeutic agent 10 is incorporated into the inner layer 36 of a vascular graft 34 according to the present invention, while a second microencapsulated chemotherapeutic agent 10 is incorporated between the layers comprising the graft 38. It is to be understood that any additional potential combinations of microencapsulated chemotherapeutic agents, alone or in combination with optional carrier agents, which may be incorporated into any layers, or spaces between the layers, of a vascular or related graft are considered within the scope of this invention.

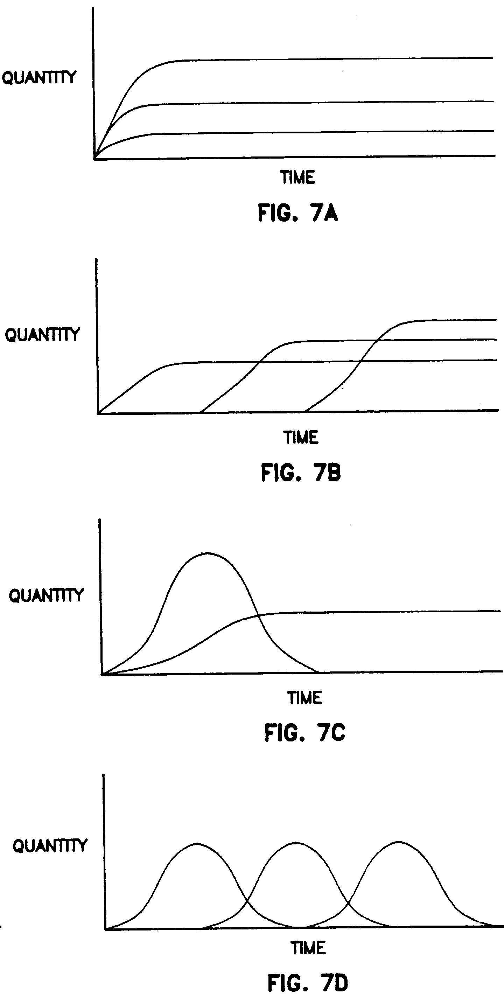

Figure 5 illustrates a cruciate ligament prosthesis 44 incorporating microencapsulated chemotherapeutic and optional carrier agents according to the method and implant of the present invention. Such prostheses are made of bundled fibers 46, composed of materials such as PTFE, and are utilized in reconstructive surgery of injured knee joints. Preferred chemotherapeutic agents include antibiotics, such as vancomycin, which help to safeguard against threatening infections, such as staph infections, which would jeopardize the implant and surrounding tissue. Also preferred are anti-inflammatory agents, such as a nonsteroidal anti-inflammatory drug, which minimize post-surgical swelling and discomfort and expedite healing and renewal of normal function.

Illustrated in Figures 6A,B, are enlarged partial sectional views through a fiber bundle 46 of the cruciate ligament prosthesis 44 shown in Figure 5, illustrating different configurations of microparticles being present. In particular, in Fig. 6A, the individual fibers 48 comprising the bundle 46 are interwoven with a matrix of chemotherapeutic and optional carrier agent encapsulating microfibers 16 or microfibrils 16 in accordance with the present invention. Alternatively, in Fig. 6B, the fibers 48 comprising the bundle 46 are surrounded by microspheres 14 incorporating chemotherapeutic and optional carrier agents.

Illustrated in Figures 7A through 7D, are various chemotherapeutic agent release patterns which might be achieved using the principles of the present invention. The charts shown illustrate quantity or dosage of the chemotherapeutic agent released over time. In Figure 7A, three separate chemotherapeutic agents are illustrated as being released at three different substantially constant levels. For example, an antibiotic, anti-inflammatory and tissue regenerative agent may all be released at varying levels at an implantation site. In Figure 7B, three different chemotherapeutic agents are released at different times. Thus, in accordance with the previous example, the antibiotic may be released first to control post-operative infection, followed by the anti-inflammatory agent to control swelling, and finally a tissue regenerative agent to aid in healing. In Figure 7C, a first chemotherapeutic agent is illustrated as being released vary early in time and then a second chemotherapeutic agent is released at a substantially constant level for a sustained period of time. An initial high dose release of an antibiotic, followed by a sustained and lower release dose of an anti-inflammatory agent would be illustrative of such a release pattern. Finally, Figure 7D illustrates three different chemotherapeutic agents being released at different times. Such an impulse release pattern may prove particularly useful with a drug which exhibits toxic effects at sustained high dosages or whose efficacy diminishes if administered continuously over a sustained period of time.

It will be appreciated that in addition to the method and implant described above, the method of the present invention may be utilized with a wide variety of other biomedical devices, including without limitation, vascular access devices, synthetic heart valve leaflets, tendon implants, transcutaneous access devices and artificial skin.

It will be further appreciated that the particular chemotherapeutic agents and optional carrier agents utilized, as well as the dosages and durations of treatment, will be in accordance with accepted treatment. The present invention addresses the manner in which the chemotherapeutic and optional carrier agents are delivered to the local treatment site.

It is to be understood, however, that even though numerous characteristics and advantages of the invention have been set forth in the foregoing description, together with details of the structure and function of the invention, the disclosure is illustrative only, and changes may be made in detail, especially in matters of shape, size and arrangement of parts within the principle of the invention, to the full extent indicated by the broad general meaning of the terms in which the appended claims are expressed.

## Claims

1. A surgical implant comprising a matrix (26) with controlled, sustained release microshapes (10) encapsulating at least one chemotherapeutic agent (12) incorporated into the matrix (26), wherein the controlled, sustained release microshapes (10) will begin to release said at least one chemotherapeutic agent (12) at a localized treatment site upon surgical implantation of the implant, **characterized** in that said matrix (26) is a non-resorbable biocompatible matrix.

2. The surgical implant of claim 1, **characterized** by further comprising at least one carrier agent incorporated into the matrix (26) of said implant so as to provide for the improved cellular uptake of said at least one chemotherapeutic agent (12).

3. The surgical implant of claim 2, **characterized** in that said carrier agent is hyaluronic acid or a derivative thereof.

4. The surgical implant of claim 2, **characterized** in that said carrier agent is encapsulated in another controlled, sustained release microshape (10).

5. The surgical implant of claim 2, **characterized** in that said carrier agent is mixed with said at least one chemotherapeutic agent (12) and encapsulated in said controlled, sustained release microshapes (10) prior to the incorporation of said controlled, sustained release microshapes into said matrix (26) of said implant.

6. The surgical implant of claim 1, **characterized** in that said implant is a vascular graft (34), vascular access device, heart valve leaflet, ligament prosthesis, tendon prosthesis, transcutaneous access device, reconstructive patch, urethral prosthesis, or artificial skin.

7. The surgical implant of claim 1, **characterized** in that said implant includes a porous polytetraflouroethylene matrix (26), and wherein the microshapes (10) are incorporated into said implant, wherein said microshapes are extruded into the matrix of said implant, said microshapes are woven in the matrix of said implant, said microshapes are mixed with a fluid or gel and are flowed into a netting or weave of said implant, said microshapes are placed between layers of said implant or positioned between said implant and an elastomer coating covering said implant.

8. The surgical implant of claim 7, **characterized** in that said fluid is hyaluronic acid and the gel is a cross-linked gel of hyaluronic acid.

## Patentansprüche

1. Chirurgisches Implantat, das eine Matrix (26) mit Mikroformen (10), in denen mindestens ein in die Matrix (26) integriertes Chemotherapeutikum (12) verkapselt ist, zur kontrollierten Dauerabgabe umfaßt, wobei die Mikroformen (10) zur kontrollierten Dauerabgabe nach chirurgischer Implantation des Implantats mit der Abgabe des mindestens einen Chemotherapeutikums (12) an einer lokalisierten Behandlungsstelle beginnen, dadurch gekennzeichnet, daß es sich bei der Matrix (26) um eine nichtresorbierbare, biokompatible Matrix handelt.

2. Chirurgisches Implantat nach Anspruch 1, dadurch gekennzeichnet, daß es weiterhin mindestens einen Träger umfaßt, der in der Matrix (26) des Implantats enthalten ist, um für die verbesserte zelluläre Aufnahme des mindestens einen Chemotherapeutikums (12) zu sorgen.

3. Chirurgisches Implantat nach Anspruch 2, dadurch gekennzeichnet, daß es sich bei dem Träger um Hyaluronsäure oder um ein Derivat davon handelt.

4. Chirurgisches Implantat nach Anspruch 2, dadurch gekennzeichnet, daß der Träger in einer anderen Mikroform (10) zur kontrollierten Dauerangabe verkapselt ist.

5. Chirurgisches Implantat nach Anspruch 2, dadurch gekennzeichnet, daß der Träger mit dem mindestens einen Chemotherapeutikum (12) vermischt und in den Mikroformen (10) zur kontrollierten Dauerabgabe verkapselt wird, bevor die Mikroformen zur kontrollierten Dauerabgabe in die Matrix (26) des Implantats eingebettet werden.

6. Chirurgisches Implantat nach Anspruch 1, dadurch gekennzeichnet, daß es sich bei dem Implantat um ein Gefäßtransplantat (34), eine Gefäßzugangsvorrichtung, ein Herzklappensegel, eine Bandprothese, eine Sehnenprothese, eine Transkutanzugangsvorrichtung, einen rekonstruktiven Lappen, eine Harnröhrenprothese oder um künstliche Haut handelt.

7. Chirurgisches Implantat nach Anspruch 1, dadurch gekennzeichnet, daß das Implantat eine poröse Polytetraflourethylenmatrix (26) enthält, und wobei die Mikroformen (10) in das Implantat eingebettet sind, wobei die Mikroformen in die Matrix des Implantats extrudiert werden, in die Matrix des Implantats gewebt werden, mit einer Flüssigkeit oder einem Gel vermischt und in ein Netz oder Gewebe des Implantats geleitet werden, zwischen Schichten des Implantats angeordnet oder zwischen dem Implantat und einem das Implantat bedeckenden elastomeren Überzug positioniert werden.

8. Chirurgisches Implantat nach Anspruch 7, dadurch gekennzeichnet, daß es sich bei der Flüssigkeit um Hyaluronsäure und bei dem Gel um ein vernetztes Hyaluronsäuregel handelt.

## Revendications

1. Implant chirurgical comprenant une matrice (26) munie de microformes (10) à libération prolongée et contrôlée encapsulant au moins un agent chimiothérapeutique (12) incorporé dans la matrice (26), dans lequel les microformes (10) à libération prolongée et contrôlée commencent à libérer ledit au moins un agent chimiothérapeutique (12) sur un site de traitement localisé lors d'une implantation chirurgicale de l'implant, caractérisé en ce que ladite matrice (26) est une matrice biocompatible non résorbable.

2. Implant chirurgical selon la revendication 1, caractérisé en ce qu'il comprend en outre au moins un agent porteur incorporé dans la matrice (26) dudit implant pour permettre la capture cellulaire améliorée dudit au moins un agent chimiothérapeutique (12).

3. Implant chirurgical selon la revendication 2, caractérisé en ce que ledit agent porteur est l'acide hyaluronique ou un dérivé de celui-ci.

4. Implant chirurgical selon la revendication 2, caractérisé en ce que ledit agent porteur est encapsulé dans une autre microforme (10) à libération prolongée et contrôlée.

5. Implant chirurgical selon la revendication 2, caractérisé en ce que ledit agent porteur est mélangé audit au moins un agent chimiothérapeutique (12) et encapsulé dans lesdites microformes (10) à libération prolongée et contrôlée avant l'incorporation desdites microformes à libération prolongée et contrôlée dans ladite matrice (26) dudit implant.

6. Implant chirurgical selon la revendication 1, caractérisé en ce que ledit implant est un greffon vasculaire (34), un dispositif d'accès vasculaire, une valve de valvule cardiaque, une prothèse ligamentaire, une prothèse tendineuse, un dispositif d'accès transcutané, un patch reconstructeur, une prothèse urétrale ou de la peau artificielle.

7. Implant chirurgical selon la revendication 1, caractérisé en ce que ledit implant comprend une matrice en polytétrafluoréthylène poreuse (26), et dans lequel les microformes (10) sont incorporées dans ledit implant, dans lequel lesdites microformes sont extrudées dans la matrice dudit implant, lesdites microformes sont tissées dans la matrice dudit implant, lesdites microformes sont mélangées à un fluide ou un gel et circulent dans un filet ou un tissage dudit implant, lesdites microformes sont placées entre les couches dudit implant ou placées entre ledit implant et un revêtement élastomère recouvrant ledit implant.

8. Implant chirurgical selon la revendication 7, caractérisé en ce que ledit fluide est l'acide hyaluronique et le gel est un gel réticulé d'acide hyaluronique.
